Europäisches Patentamt

European Patent Office

Office européen des brevets

(19)

(11) **EP 0 973 544 B1**

(12) **EUROPÄISCHE PATENTSCHRIFT**

(45) Veröffentlichungstag und Bekanntmachung des Hinweises auf die Patenterteilung:
**27.06.2001 Patentblatt 2001/26**

(21) Anmeldenummer: **98913425.9**

(22) Anmeldetag: **06.04.1998**

(51) Int Cl.⁷: **A61K 38/48**

(86) Internationale Anmeldenummer:
**PCT/AT98/00091**

(87) Internationale Veröffentlichungsnummer:
**WO 98/44942 (15.10.1998 Gazette 1998/41)**

(54) **IMMUNTOLERANTE PROTHROMBINKOMPLEX-PRÄPARATION**

IMMUNOTOLERANT PROTHROMBIN COMPLEX PREPARATION

PREPARATION IMMUNOTOLERANTE CONTENANT UN COMPLEXE DE PROTHROMBINE

(84) Benannte Vertragsstaaten:
**AT BE CH CY DE DK ES FI FR GB GR IE IT LI LU MC NL PT SE**
Benannte Erstreckungsstaaten:
**SI**

(30) Priorität: **08.04.1997 AT 59497**
**19.09.1997 AT 159297**

(43) Veröffentlichungstag der Anmeldung:
**26.01.2000 Patentblatt 2000/04**

(73) Patentinhaber: **Baxter Aktiengesellschaft**
**1221 Wien (AT)**

(72) Erfinder:
• **SCHWARZ, Hans-Peter**
**A-1180 Wien (AT)**
• **TURECEK, Peter**
**A-3400 Klosterneuburg (AT)**

(74) Vertreter: **Alge, Daniel, Mag. Dr. rer.nat. et al Patentanwälte**
**Sonn, Pawloy, Weinzinger & Köhler-Pavlik**
**Riemergasse 14**
**1010 Wien (AT)**

(56) Entgegenhaltungen:
**EP-A- 0 765 669**          **WO-A-81/02105**
**WO-A-83/00016**          **GB-A- 2 080 312**

## Beschreibung

Die Erfindung betrifft eine pharmazeutische Zusammensetzung zur Behandlung von Blutgerinnungsstörungen, insbesondere von Faktor VIII Inhibitor-Patienten. Die Erfindung betrifft weiters ein Verfahren zur Herstellung einer derartigen Zusammensetzung sowie deren Verwendung.

Der Gegenstand der Erfindung wird in den Ansprüchen 1-7 angegeben.

Die Blutgerinnung wird durch eine Reihe von aufeinanderfolgenden Reaktionen verschiedener Proteine bzw. Enzyme ausgelöst. Durch einen Mangel an Blutgerinnungsfaktoren wird die Bildung von Fibrin aus Fibrinogen und damit der Wundverschluß verhindert; die Folge sind Blutungen. Ein solcher Fall liegt bei der Hämophilie A vor. Diese ist die am meisten verbreitete Blutungskrankheit und wird durch den Mangel an Faktor VIII hervorgerufen. Zur Substitutionsbehandlung der Hämophilie A werden Präparate eingesetzt, die den Faktor VIII enthalten. Die Behandlung mit diesen Präparaten führt in den meisten Fällen zu einer raschen Blutstillung.

Es gibt jedoch auch Patienten, bei denen nicht nur ein Mangel an Faktor VIII auftritt, sondern die auch einen gegen Faktor VIII gerichteten Hemmstoff (Inhibitor) entwickelt haben. Ein weiteres Patientenkollektiv weist Faktor VIII-Inhibitoren auf, ohne an Hämophilie A zu leiden. Je nach vorhandener Menge an Faktor VIII-Inhibitoren wird die Wirkung von zugeführtem Faktor VIII durch dessen Neutralisierung inhibiert.

Zur Behandlung von Faktor VIII-Inhibitor-Patienten werden derzeit Präparate auf Basis einer Plasmafraktion angeboten, die ein Gemisch von Gerinnungsfaktoren enthält. Diese Plasmafraktion kann beispielsweise die Faktoren des Prothrombinkomplexes (Faktor II, VII, IX und X) enthalten. Eine blutgerinnungsfördernde Präparation mit Faktor VIII-Inhibitor-Bypass-Aktivität (FEIBA® TIM 4, Fa. IMMUNO AG) wird beispielsweise gemäß der AT-B 0 368 883 durch eine Behandlung von Kryoüberstand erhalten. Diese Präparation enthält ebenfalls die Gerinnungsfaktoren II, VII, IX und X.

Die Wirkung eines FEIBA-Präparates ist aufgrund dessen komplexer Zusammensetzung vielschichtig. Mariani et al. (Thrombosis Res. 31, 475-488 (1983)) nennt als ein Wirkungsprinzip den Faktor VII in seiner aktivierten Form. Es wurde festgestellt, daß nach Infusion einer FEIBA-Präparation ein erhöhter Gehalt von Faktor VIIa im Plasma Hämophiler auftritt.

Ebenso wird von Teitel (Thrombosis and Haemostasis 66 (5) 559-564, (1991)) die Rolle von Faktor VIIa in Prothrombinkomplex-Konzentraten mit einer "Faktor VIII-Bypassing-Aktivity" diskutiert. Gleichzeitig wird auch auf das Wirkungsprinzip von Faktor Xa in derartigen Präparaten eingegangen. Die untersuchten Prothrombinkomplex-Konzentrate enthielten Faktor VIIa, ausgedrückt durch das Verhältnis der Faktor VII-Aktivität zu Faktor VII-Antigen von 2,1 und 2,5.

Die gemäß der EP 0 044 343-B1 hergestellte Prothrombin-haltige therapeutische Zusammensetzung ist zur Behandlung von Gerinnungsfaktor-Inhibitoren geeignet und enthält einen aktivierten Prothrombinkomplex, bei dem die Faktoren teilweise aktiviert sind. Der Anteil an Faktor VIIa liegt bei 8-80 Einheiten/ml. Die Faktor IX-Konzentration liegt im Bereich von 15 bis 112 Einheiten/ml. Entsprechend beträgt der Gehalt an Faktor VIIa, bezogen auf Faktor IX, 0,07 - 5,3 E Faktor VIIa/E Faktor IX. Vinazzer (Thromb. Res. 26:21-29 (1982)) zeigt den Unterschied der Präparate AUTOPLEX, welches gemäß der EP 0 044 343 hergestellt wird, und FEIBA. Wie dort gezeigt wird, zeichnet sich AUTOPLEX durch den höheren Gehalt an Thrombin (Faktor IIa), gemessen in NIH-Units, im Vergleich zu FEIBA aus (siehe Tabelle 1, Seite 24).

Aber auch hochgereinigte Faktor VIIa-Präparationen werden zur Therapie von Gerinnungsfaktor-Inhibitorzuständen vorgeschlagen (z.B. EP 0 082 182-B1) und Hedner et al. (Haemostasis 19, 335-343 (1989)).

Ein Vorteil von Faktor VIIa-Präparationen ist deren Freiheit an Faktor VIII. Der Gehalt an Faktor VIII in Prothrombinkomplex-Präparaten oder in aktivierten Prothrombinkomplex-Präparaten wie z.B. FEIBA bewirkt nämlich in Patienten mit funktionellen Faktor VIII-Inhibitoren, daß diese inhibierenden Antikörper durch neuerliche Gabe von Faktor VIII geboostert werden, womit sich der Zustand der Inhibitor-Hämophilie vorübergehend sogar verschlechtert.

Es wurde allerdings festgestellt, daß zur effektiven Blutstillung bei der Faktor VIII-Inhibitor Hämophilie Faktor VIIa-Präparationen in ihrer Wirkung den Prothrombinkomplexfaktoren-Präparaten unterlegen sind (Turecek P. et al., Thrombosis & Haemostasis, 1997, p.222).

Eine Aufgabe der vorliegenden Erfindung ist es daher, eine Präparation zur Verfügung zu stellen, die die Effektivität bzw. Effizienz von Prothrombinkomplexfaktoren-Präparaten besitzt, ohne jedoch die unerwünschten immunologischen Nebenreaktionen derartiger Präparationen aufzuweisen.

Die vorstehend genannte Aufgabe wird dadurch gelöst, daß gemäß der vorliegenden Erfindung eine immuntolerante pharmazeutische Prothrombinkomplex-Präparation enthaltend die Faktoren II, IX, X und gegebenenfalls VII mit einem geringen Faktor VIII-Antigengehalt zur Verfügung gestellt wird.

Der Faktor VIII-Antigengehalt der erfindungsgemäßen Präparation liegt vorzugsweise bei weniger als 10%, insbesondere weniger als 5%. Der Faktor VIII-Gehalt ist kleiner als 0,1 Faktor VIII:C Antigen/E FEIBA. In einer bevorzugten Ausführungsform liegt der Faktor VIII Antigengehalt sogar unter 0,03/E FEIBA, noch bevorzugter unter 0,02/E FEIBA und am meisten bevorzugt unter der Nachweisgrenze.

Der Befund, daß insbesondere bei einer weiteren Reinigung der Prothrombinkomplexfaktoren aus Plasma oder einer Plasmafraktion, der Faktor VIII-Gehalt und gegebenenfalls auch der Phospholipid-Gehalt derart reduziert werden kann, während die Aktivität der Prothrombinkomplexfaktoren weitgehend erhalten bleibt, ist als überraschend anzusehen.

Insbesondere enthält die erfindungsgemäße pharmazeutische Präparation mindestens die Faktoren IXa, Xa und VIIa und weist FEIB-Aktivität auf, verkürzt also die Gerinnungszeit eines Faktor VIII-defizienten Plasmas mit einem funktionellen Inhibitor (siehe dazu beispielsweise die AT-B 350 726).

Eine erfindungsgemäße Präparation kann aus Plasma oder einer Plasmafraktion hergestellt werden. Die Plasmafraktion kann aus Plasma, insbesondere Plasma humanen Ursprungs, durch eine chromatographische Behandlung, Fällung oder Zentrifugation hergestellt werden, oder es wird der Kryopräzipitatüberstand verwendet.

Die Plasmafraktion enthält Vitamin-K-abhängige Faktoren wie Faktoren des Prothrombin-Komplexes, aber auch Protein S, C und/oder Z sind vorzugsweise enthalten.

In einer besonders bevorzugten Ausführungsform ist die Präparation frei von Phospholipiden. Vorzugsweise liegt die Obergrenze enthaltener Phospholipide bei 0,1 nmol/E FEIBA (zur Bestimmung siehe Beispiele).

Aufgrund dieser Freiheit von Phospholipiden, kann die Bildung unerwünschter Antikörper gegen Faktor VIII vermindert bzw. verhindert werden.

Gemäß der vorliegenden Erfindung wird auch ein Verfahren zur Herstellung dieser Präparation zur Verfügung gestellt. Dieses Verfahren umfaßt die folgenden Schritte:

a) zur Verfügung Stellen von Plasma oder einer Plasmafraktion enthaltend die Faktoren II, IX, X und gegebenenfalls VII,

b) in Kontakt Bringen des Plasmas oder der Plasmafraktion mit einem Trägermaterial, gegebenenfalls in Gegenwart eines Detergens, so daß Faktor VIII und gegebenenfalls Phospholipide von den Faktoren II, IX, X und gegebenenfalls VII getrennt werden,

c) Reinigen des Plasmas oder der Plasmafraktion, und

d) Gewinnen einer Fraktion enthaltend die Faktoren II, IX, X und gegebenenfalls VII.

In einer bevorzugten Variante werden die Schritte b und c bzw. b, c und d als ein Verfahrensschritt durchgeführt.

Bei der verwendeten Plasmafraktion handelt es sich vorzugsweise um eine mit wenigstens intermediärer Reinheit.

Unter einer Präparation mit intermediärer Reinheit ist eine derartige Plasmafraktion zu verstehen, welche der Definition der intermediären Reinheit von Faktor VIII-Präparaten analog ist (siehe hierzu beispielsweise Wood Clive (ed.), Factor VIII: Purity and prophylaxis, Royal Society of Medicine).

Begleitproteine, die durch eine chromatographische Reinigung nicht entfernt werden, können somit bei einem Präparat intermediärer Reinheit noch vorhanden sein.

Als Ausgangsmaterial kann auch eine herkömmliche, am Markt erhältliche Prothrombin-Komplex-Faktoren-Präparation verwendet werden, wie z.B. FEIBA S-TIM 4, IMMUNO oder aktivierter Prothrombinkomplex.

Als entsprechende Reinigungsverfahren kommen die aus dem Stand der Technik bekannten Methoden zum Einsatz, bevorzugterweise wird eine chromatographische Behandlung, Fällung oder Zentrifugation durchgeführt. Als Plasmafraktion kann auch Kryopräzipitatüberstand verwendet werden.

Das Trägermaterial ist ein für die Chromatographie, Filtration und/oder Nanofiltration geeignetes Material. Bei der Filtration handelt es sich insbesondere um eine Affinitäts- oder Membranfiltration.

Wird ein vorgereinigtes Material verwendet, beispielsweise ein mittels Anionenaustauscher vorgereinigtes Material, so kann eine Readsorption an einem weiteren Trägermaterial, vorzugsweise an demselben Trägermaterial wie für die Vorreinigung verwendet, unter geänderten Bedingungen erfolgen.

In einer bevorzugten Ausführungsform ist das Trägermaterial ein für Faktor VIII spezifisches Trägermaterial, insbesondere eine für die Affinitätschromatographie geeignete Matrix. Besonders bevorzugt wird eine vWF-hältige Matrix verwendet.

An ein derartiges Trägermaterial wird Faktor VIII und gegebenenfalls Phospholipid bevorzugt adsorbiert, während die Faktoren II, IX, X und gegebenenfalls VII nicht gebunden werden.

Das Trägermaterial kann aber auch ein für Faktor VIII unspezifisches Trägermaterial sein, beispielsweise ein schwacher Anionenaustauscher, beispielsweise ein DEAE-, TMAE- oder weitere aus dem Stand der Technik hinreichend bekannte Anionenaustauscher.

Je nach gewählten Bedingungen wird entweder Faktor VIII und gegebenenfalls Phospholipide an dem Trägermaterial adsorbiert, während die Faktoren II, IX, X und gegebenenfalls VII nicht gebunden werden oder umgekehrt.

In einer weiteren bevorzugten Ausführungsform weist das Trägermaterial eine für den Prothrombinkomplex höhere Affinität auf als für den Faktor VIII. So werden beispielsweise die Faktoren II, IX, X und gegebenenfalls VIII adsorbiert, während Faktor VIII und gegebenenfalls Phospholipide eluiert werden.

Der Faktor VIII kann auch gezielt inaktiviert werden und dann in dieser inaktivierten Form beispielsweise nicht mehr an das Trägermaterial binden. Eine derartige Inaktivierung des Faktor VIII kann beispielsweise durch Dissoziation unter Verwendung von z.B. einem Chelatbildner, durch Abbau, insbesondere proteolytischen Abbau, z.B. durch Serinproteasen wie Thrombin oder aktiviertem Protein C, durch Binden von Affinitätspartnern, wie Antikörper oder Peptiden, erreicht werden.

Alle beschriebenen Verfahrensvarianten können auch in Gegenwart eines Detergens, insbesondere eines nicht-ionischen Detergens, durchgeführt werden. Vorzugsweise wird als Detergens ein Polyether oder ein Polysorbat verwendet, insbesondere wird Tween oder Triton verwendet.

Ist Detergens vorhanden, wird es in einer bevorzugten Ausführungsform wieder entfernt bzw. abgetrennt.

In einer weiteren bevorzugten Ausführungsform ist ein Schritt zur Inaktivierung von gegebenenfalls vohandenen Pathogenen, insbesondere einer ausgewählt aus der Gruppe Hitzebehandlung, Dampfbehandlung, Behandlung mit einem Lösungsmittel und/oder Behandlung mit einem Detergens, vorgesehen.

Die erfindungsgemäße Präparation ist also allgemein erhältlich nach einem vorstehend beschriebenen Verfahren. Sie eignet sich insbesondere zur Herstellung eines Medikaments, welches geeignet ist für die Behandlung von Faktor VIII-Inhibitor (= Haemophilie A) Patienten, insbesondere für solche Patienten mit einem Inhibitor-Titer von größer 1 Bethesda E/ml Plasma, vorzugsweise größer 5 Bethesda E/ml Plasma. Sie kann auch durch Kombination der hochgereinigten Einzelfaktoren II, IX und X sowie gegebenenfalls VII hergestellt werden.

Da biologisches Material, also Material, das von Organismen bzw. Körperflüssigkeiten oder Mikroorganismen stammt, mit Pathogenen, wie z.B. infektiöse Moleküle oder Mikroorganismen und Viren, bzw. Pyrogenen, kontaminiert sein kann, wurden bereits verschiedene Verfahren zur Inaktivierung bzw. Abreicherung von Pathogenen bzw. Pyrogenen entwickelt.

Derartige Verfahren beinhalten physikalische und/oder chemische Behandlungen, wie beispielsweise diverse Filtrationsmethoden (z.B. Nano-, Dia- oder Ultrafiltration), Hitzebehandlung, Behandlung mit Säure oder Lauge, Behandlung mit Detergens und/oder organisches Lösungsmittel sowie Behandlung mit UV-Licht oder mit Laser-Licht. Auch verschiedene Kombinationen solcher Verfahren zur Inaktivierung bzw. Abreicherung von Pathogenen wurden im Stand der Technik vielfach vorgeschlagen.

Aus der EP 0 197 554 ist beispielsweise ein Verfahren zum Depyrogenisieren und Inaktivieren von Viren in einem biologischen oder pharmazeutischen Produkt bekannt, welches eine Behandlung mit einem virusinaktivierenden und depyrogenisierenden Mittel, wie z.B. eine amphiphile Substanz und/oder ein Lösungsmittel, an einer festen Phase, an der das Produkt adsorbiert vorliegt, umfaßt. Nach dieser Behandlung wird das virusinaktivierende und depyrogenisierende Mittel von der festen Phase abgetrennt, das adsorbierte Produkt gewaschen und schließlich von der festen Phase eluiert.

Aus der EP 0 131 740 ist die Behandlung einer Protein enthaltenden Zusammensetzung in einer Lösung mit organischen Lösungsmitteln wie Di- oder Trialkylphosphaten, gegebenenfalls in Gegenwart eines Detergens (Solvens/Detergens-Behandlung) bekannt, wodurch Protein-Zusammensetzungen frei von lipidhältigen Viren erhalten werden können.

Aus der AT-PS 402 151 ist eine Hitzebehandlung bekannt, bei welcher einem in wäßriger Lösung vorliegenden Präparat vor dem Erhitzen ein Tensid in einer Konzentration von mindestens 1 Gew.% zugesetzt wird.

Ein weiteres Verfahren zur Reduktion bzw. Suppression unerwünschter Aktivitäten in biologischen oder pharmazeutischen Produkten ist aus der EP 0 083 999 bekannt. Dieses basiert auf einem verlängerten Kontakt mit einer Lösung oder Suspension eines nicht-denaturierend wirkenden Amphiphils. Das depyrogenisierte Produkt wird zur Entfernung des Amphiphils mit einem Ionenaustauscher behandelt.

Ein Nachteil vieler dieser aus dem Stand der Technik bekannten Verfahren ist das häufige Auftreten von Aktivitätsverlusten der in den zu behandelnden Zusammensetzungen enthaltenen labilen Proteine, beispielsweise von Blutproteinen. Insbesondere bei Durchführung eines chromatographischen Reinigungsschrittes erfolgt eine Inaktivierung von Proteinen in einem relativ hohen Ausmaß. Ein Abbau von Proteinen kann auch zu einer Aktivierung führen. So ist beispielsweise bekannt, daß der Faktor VII bei einer chromatographischen Reinigung aufgrund autokatalytischer Vorgänge sehr leicht zu unerwünschtem, weil sehr labilen, Faktor VIIa aktiviert wird.

Ein weiterer Nachteil liegt in dem hohen zeitlichen und apparativen Aufwand vieler Verfahren, was deren Praktikabilität stark mindert und deshalb die Anwendung im großtechnischen Maßstab häufig ungeeignet macht.

Im Rahmen der vorliegenden Erfindung soll daher ein für Proteine, insbesondere für labile Blutproteine, schonendes Verfahren zur wirksamen Inaktivierung von Pathogenen in biologischen Materialien verwendet werden, welches auch leicht in einen großtechnischen Maßstab übertragbar ist und ökonomisch durchgeführt werden kann. Insbesondere soll bei dem Verfahren zur Inaktivierung von Pathogenen ein Abbau und eine mögliche Aktivierung hierfür empfindlicher Proteine weitgehend vermieden werden.

Bei diesem Verfahren zur Inaktivierung von Pathogenen, insbesondere von Viren, in einem biologischen Material wird dieses Material mit einem chemischen Mittel inkubiert, wobei die Inkubation in Gegenwart eines eluotropen Salzes entsprechend einer NaCl-Konzentration von mindestens 200 mmol/l, vorzugsweise mindestens 300 mmol/l, vorgenommen wird.

Die Inaktivierung von Pathogenen in Lösung bietet gegenüber der Behandlung eines Adsorbens einige Vorteile. So ist beispielsweise die Praktikabilität eines derartigen Verfahrens in einem homogenen, einphasigen System höher und die Validierung des Inaktivierungsschrittes besser möglich. Auch scheint die bessere Zugänglichkeit von Pathogenen in einer relativ homogenen Phase die Effizienz des Verfahrensschrittes zu erhöhen.

Das biologische Material enthält vorzugsweise ein humanes Protein und ist insbesondere Plasma oder eine Plasmafraktion oder stammt von einer Zellkultur. Vorzugsweise enthält das biologische Material einen Blutfaktor, wie Faktor XII, XI, VIII, V, von Willebrand-Faktor oder Fibrinogen, insbesondere ein Vitamin-K-abhängiges Protein wie Faktor II, Faktor VII, Faktor IX, Faktor X, Protein C, Protein S bzw. Protein Z.

Die Proteine können als Einzelfaktoren, vorzugsweise in gereinigter Form, oder in einem komplexen Gemisch vorliegen. In einer ganz besonders bevorzugten Ausführungsform enthält das biologische Material mindestens einen Faktor des Prothrombinkomplexes und ist insbesondere eine Prothrombinkomplex enthaltende Fraktion oder ein Faktor VII enthaltendes Material, beispielsweise wird nach einer Kryopräzipitation von Plasma vom entsprechenden Überstand (Kryoüberstand) ausgegangen.

Die erfindungsgemäße Präparation ist bevorzugterweise eine solche mit FEIB-Aktivität (Factor Eight Inhibitor Bypassing-Activity), also eine Präparation, die zur Behandlung von Faktor VIII-Inhibitor-Patienten geeignet ist.

Das von einer Zellkultur stammende Material ist vorzugsweise ein Material, enthaltend rekombinant hergestellte Blutfaktoren, darunter Faktoren der intrinsischen oder extrinsischen Gerinnung, der Fibrinolyse, der Thrombolyse oder deren Inhibitoren, insbesondere Vitamin-K-abhängige Blutfaktoren. Als Zellen kommen die für die Expression rekombinanter Proteine gängigen Zellen in Frage, bevorzugterweise Säugerzellen, wie beispielsweise Vero-, CHO- oder BHK-Zellen. Die entsprechenden Proteine können direkt aus dem rohen Zellextrakt dem erfindungsgemäßen Verfahren zur Inaktivierung gegebenenfalls vorhandener Pathogene unterworfen werden, es kann sich jedoch auch um eine vorgereinigte Zellfraktion handeln.

Das chemische Mittel ist beispielsweise ein Detergens (Amphiphil, Tensid), welches vorzugsweise in einer Menge von mindestens 1%, mehr bevorzugt mehr als 5%, am meisten bevorzugt mehr als 10% enthalten ist; es können aber auch andere chemische Mittel erfindungsgemäß eingesetzt werden, insbesondere solche, für die z.B. eine viruzide, bakterizide oder depyrogenisierende Wirkung bereits bekannt ist, bzw. Mischungen verschiedenster chemischer Mittel.

Die Auswahl ist jedoch dadurch limitiert, daß die Nativität des biologischen Materials nicht wesentlich beeinträchtigt werden soll. Für eine ökonomische Verfahrensweise wird eine Chemikalie gewählt, die die biologische Aktivität des Materials zu mehr als 50% erhält, bezogen auf die Aktivität vor dem Inkubieren, vorzugsweise mindestens 70%, insbesondere mehr als 85%. Erhalt der biologischen Aktivität bedeutet, daß die im biologischen Material enthaltenen Proteine die ihnen natürlicherweise zugeschriebene Funktion bzw. die verschiedenen Funktionen ausüben können. Diese biologische Aktivität kann dann je nach Art des Proteins ermittelt und angegeben werden, beispielsweise mittels eines standardisierten chromogenen Tests oder der Antigen-Bestimmung. Gegebenenfalls wird das chemische Mittel nach der Inkubation abgetrennt.

Unter einem Detergens ist allgemein eine synthetische, organische, grenzflächenaktive Substanz zu verstehen.

Bevorzugterweise wird bei dem erfindungsgemäßen Verfahren ein nichtionisches Detergens verwendet. Nichtionische Tenside wie Polyether, insbesondere Alkylphenolpolyglykolether, sind u.a. Produkte der Ethoxylierung von Fettsäuren, Fettsäureamiden, Fettaminen, Fettalkoholen, Aminoxiden, Fettsäureestern von Polyalkoholen und Zuckerestern.

Ein solches Tensid wirkt auf die Proteine nicht denaturierend und ist bevorzugterweise ausgewählt aus der Gruppe Polysorbat und Triton. Als Polysorbat wird beispielsweise Tween® verwendet.

Wenn Detergentien als chemische Mittel eingesetzt werden, so werden diese gemäß einer bevorzugten Ausführungsform ohne den Zusatz anderer Mittel eingesetzt, insbesondere ohne den Zusatz von toxischen organischen Substanzen oder Lösungsmitteln, wie z.B. TNBP. Dadurch wird ein Kontaminationsrisiko minimiert.

Das biologische Material wird gemäß dem erfindungsgemäßen Verfahren mit einem chemischen Mittel inkubiert. Inkubation bedeutet das In-Kontakt-bringen des biologischen Materials mit einer Lösung, Suspension oder Emulsion eines chemischen Mittels für einen zur Inaktivierung gegebenenfalls vorhandener Pathogene bzw. Pyrogene ausreichend langen Zeitraum bei einer bestimmten Temperatur. Das In-Kontakt-bringen kann beispielsweise einfach durch Stehenlassen des Gemisches für einen definierten Zeitraum erfolgen.

Die Inkubation erfolgt gemäß der vorliegenden Erfindung in Gegenwart eines eluotropen Salzes. Unter "eluotropem Salz" ist im folgenden das Salz in Gemisch mit chemischen Mittel oder das Salz in einer komplexen Zusammensetzung zu verstehen, mit der Eigenschaft, adsorbierte Stoffe aus festen oder mit Flüssigkeit getränkten, auch gelartigen Adsorbentien herauszulösen und/oder zu verdrängen. Bevorzugterweise handelt es sich bei dem eluotropen Salz um ein Desorptionsmittel, wie es bei chromatographischen Verfahren zur Anwendung kommt. Der adsorbierte Stoff ist i.a. in Gegenwart des eluotropen Salzes ausreichend löslich, d.h. es werden vorzugsweise Bedingungen gewählt, die das biologische Material nicht präzipitieren.

Die Art und Konzentration des Salzes bzw. der Zusammensetzung wird im allgemeinen je nach verwendetem Adsorbens gewählt. Die eluierende Wirkung eines Salzes hängt beispielsweise von der Polarität des Lösungsmittels ab, nimmt also z.B. in der Reihenfolge Ethanol - Aceton - Methanol - Wasser zu. Das Adsorbens kann eine feste Phase sein, insbesondere eine für die Ionenaustausch-Chromatographie geeignete Matrix. In der das eluotrope Salz enthaltenden Zusammensetzung können auch weitere Zusätze, beispielsweise weitere Salze, enthalten sein. Vorzugsweise handelt es sich bei der Zusammensetzung um eine wäßrige Zusammensetzung mit einem pH-Wert im Bereich zwischen 6,0 bis 8,0, bevorzugterweise um 7,0.

In einer bevorzugten Ausführungsform wird als eluotropes Salz Natriumchlorid verwendet, aber auch andere Alkali- oder auch Erdalkalisalze, darunter $CaCl_2$. Als eluotrope Salze können auch sogenannte Chaotrope, wie z.B. Harnstoff, Rhodanide oder Guanidin, eingesetzt werden. Die Konzentration des Salzes ist mindestens $\geq$ 200 mmol/l, vorzugsweise $\geq$ 300 mmol/l. Die obere Grenze für die eingesetzte Konzentration hängt insbesondere von der Löslichkeit des jeweiligen Salzes ab und liegt für NaCl beispielsweise um 2 mol/l. Chaotrope Substanzen, wie z.B. Harnstoff, können gegebenenfalls sogar bis zu einer Konzentration von 8 mol/l eingesetzt werden.

Die Inkubation des biologischen Materials mit dem chemischen Mittel erfolgt für einen für die Inaktivierung gegebenenfalls vorhandener Pathogene ausreichend langen Zeitraum, vorzugsweise für einen Zeitraum zwischen 10 Minuten und 10 Stunden, am meisten bevorzugt zwischen 1 Stunde und 5 Stunden. Der benötigte Zeitraum für das erfindungsgemäße Verfahren kann mittels Modellviren wie HIV, Sindbis-, FSME- oder Hepatitis-Viren in einem Vorversuch bestimmt werden.

Auch die Wahl der Temperatur beeinflußt den anzuwendenden Zeitraum. In dem erfindungsgemäßen Verfahren wird bevorzugterweise bei Raumtemperatur, beispielsweise in einem Temperaturbereich zwischen 15 und 45°C, insbesondere zwischen 20 und 30°C, inkubiert.

Beim erfindungsgemäßen Verfahren wird das biologische Material bevorzugt an einem festen Träger adsorbiert, gereinigt und die Inkubation unmittelbar nach Elution des gereinigten Materials vorgenommen. Elution und Inkubation können konsekutiv durchgeführt werden, sie können aber auch gleichzeitig erfolgen.

Gemäß einer weiteren bevorzugten Ausführungsform wird die Inkubation nach einer chromatographischen Reinigung eines biologischen Materials vorgenommen, wobei das Eluat noch weiter prozessiert wurde, beispielsweise durch Zentrifugation, Filtration oder andere physikalische Methoden.

Der feste Träger ist bevorzugterweise ein für die Chromatographie geeignetes Material, insbesondere ein für die Ionenaustausch-Chromatographie, hydrophobe Chromatographie oder die Affinitätschromatographie geeignetes Material. Beispielsweise werden Materialien wie Sepharose® , Superdex, Sephadex® , Spherodex® , Toyopearl® , oder anorganische Materialien, wie Hydroxylapatit, verwendet.

Als Ionenaustauscher können Anionenaustauscher-Materialien, wie beispielsweise DEAE-Sephacel® , DEAE-Sephadex® , DEAE-Sepharose® CL6B, DEAE-Sepharose® Fast Flow, QAE-Sephadex® , Q-Sepharose® Fast Flow, Q-Sepharose® High Performance, DEAE-Tris-Acryl, DEAE-Spherodex® , Q-Hyper-D (erhältlich durch die Firma Sepracor), DEAE-Toyopearl® , QAE-Toyopearl® , Fractogel® EMD-TMAE oder andere Fractogel-Material verwendet werden.

Als Beispiele für hydrophobe Chromatographiematerialien sollen beispielsweise Butyl-Sepharose® , Octyl-Sepharose® , Phenyl-Sepharose® , Fractogel® TSK-Butyl, t-Butyl-HIC Support oder TSK-Gel Butyl Toyopearl® genannt werden.

Das biologische Material kann direkt aus einem komplexen Gemisch an den Träger adsorbiert und gereinigt werden, dem Inaktivierungsschritt können aber auch weitere Schritte zur Reinigung des Materials vor- oder nachgeschaltet werden, wobei im Rahmen der vorliegenden Erfindung weitere chromatographische Reinigungsschritte bevorzugt werden.

Durch das erfindungsgemäße Verfahren werden Pathogene inaktiviert. Unter Pathogenen werden auch Bruchstücke von z.B. Viren, insbesondere auch das isolierte Genom oder dessen Fragmente, verstanden.

Die Pathogene können lipidumhüllte Pathogene, wie z.B. Hepatitis B-Virus oder nicht-lipidumhüllte Pathogene, wie z.B. Hepatitis A-Virus, sein.

Virusinaktivierungsverfahren werden heutzutage als wirksam bezeichnet, wenn nach Anwendung des Verfahrens an einer Probe eines biologischen Materials, welche mit einer hohen Dosis eines Testvirus, z.B. HI-Virus oder Sindbis-Virus als Modellvirus für Hepatitis-Viren, versetzt wurde, keine Viren mehr in der Probe nachgewiesen werden können und der Virustiter somit unter die Nachweisgrenze reduziert wurde. Nachweis und Quantifizierung von Nukleinsäuren kann beispielsweise mittels einer PCR-Methode, wie in der AT-PS 401 062 beschrieben, erfolgen oder durch direkte Titration.

Als Maß für die Inaktivierung ist der sogenannte Reduktionsfaktor bekannt, der nach einer eimaligen Zugabe von Testvirus aus dem dekadischen Logarithmus des Quotienten von Anfangs- und Endvirustiter errechnet wird. Aus der Europäischen Richtlinie EC III/8115/89-EN der Kommission der Europäischen Gemeinschaften ist weiters der sogenannte Gesamt-Reduktionsfaktor bekannt. Er wird aus der Summe der Reduktionsfaktoren von einzelnen subsequenten Inaktivierungsmaßnahmen errechnet.

Auch ein weiterer unabhängiger Schritt zur Inaktivierung bzw. Abreicherung von Pathogenen wird vorzugsweise durchgeführt. Hierfür kommen alle aus dem Stand der Technik bekannten Verfahren in Frage, um das Infektionsrisiko zu minimieren.

Insbesondere wird als ein weiterer Schritt zur Inaktivierung bzw. Abreicherung eine Filtration und/oder eine Hitzebehandlung vorgenommen.

Als Filtration wird vorzugsweise eine Nanofiltration vorgenommen. Eine bevorzugte Hitzebehandlung wird am festen biologischen Material durchgeführt, z.B. an einem Lyophilisat mit kontrolliertem Wassergehalt, beispielsweise einem Wassergehalt zwischen 5 bis 8% und einer Temperatur zwischen 50 und 80°C, wie in der EP-0 159 311 beschrieben.

In einer bevorzugten Ausführungsform ist eine 2-stufige Behandlung mit einem Detergens als chemischem Mittel vorgesehen. Dabei wird in einem ersten Schritt ein Detergens in einer Menge von wenigstens 1%, vorzugsweise wenigstens 5%, am meisten bevorzugt wenigstens 10% verwendet. In einer zweiten Stufe wird ein weiteres Detergens in einer Menge von mindestens 10%, vorzugsweise mindestens 12%, am meisten bevorzugt mindestens 14% verwendet. Das verwendete Detergens kann für beide Stufen dasselbe sein, es können aber auch unterschiedliche Detergentien eingesetzt werden. Ganz allgemein kann durch die Kombination von Schritten zur Virusinaktivierung das Risiko einer Virusinfektion nach Verabreichung einer entsprechenden Präparation stark reduziert bzw. ausgeschlossen werden.

Gemäß der vorliegenden Erfindung wird auch eine chromatographisch gereinigte Präparation zur Verfügung gestellt, enthaltend einen autodynamisch aktivierbaren Blutfaktor mit einem Anteil an aktiviertem Blutfaktor von weniger als 50%, bezogen auf den Gehalt an dem aktivierten und nicht aktivierten Blutfaktor, vorzugsweise weniger als 40%, mehr bevorzugt weniger als 30%, noch mehr bevorzugt weniger als 20%, weiters bevorzugt weniger als 10%, am meisten bevorzugt weniger als 1%, und einem Detergens-Gehalt.

Insbesondere handelt es sich bei der Präparation um eine einen Prothrombinkomplex enthaltende Präparation mit einer Faktor VIIa-Aktivität von weniger als 50%, bezogen auf den Gehalt an aktiviertem und nicht aktiviertem Faktor VII, vorzugsweise weniger als 10%, am meisten bevorzugt weniger als 1%. Der Detergensgehalt des erfindungsgemäßen Präparates liegt dabei in einer pharmazeutisch akzeptablen Menge vor, vorzugsweise zwischen 1% und der Nachweisgrenze des Detergens.

Unter einem autodynamisch aktivierbaren Blutfaktor ist gemäß der vorliegenden Erfindung ein Blutfaktor zu verstehen, der autokatalytisch, durch Oberflächenkontakt oder durch Prozesse, wie beispielsweise chromatographische Prozesse, aktivierbar ist. Insbesondere ist ein derartiger Blutfaktor ein solcher, ausgewählt aus der Gruppe Faktor VII, Faktor XII, Faktor XI und Prä-Kallikrein.

In einer weiteren bevorzugten Ausführungsform ist die Präparation frei von Serinprotease-Inhibitoren, wie beispielsweise Thrombin-Inhibitoren, bzw. der Kofaktoren, wie z.B. Heparin. In einer speziellen Ausführungsform ist die Freiheit von derartigen Substanzen bereits während eines chromatographischen Prozesses gegeben.

Daher betrifft die vorliegende Erfindung auch entsprechende Präparationen, erhältlich durch das erfindungsgemäße Verfahren.

In der erfindungsgemäßen Präparation können auch weitere Zusätze enthalten sein, beispielsweise stabilisierend wirkende Substanzen wie Aminosäuren.

Die nachstehenden Beispiele soll die vorliegende Erfindung näher erläutert werden, ohne diese allerdings darauf zu beschränken.

**Beispiel 1 : Bestimmung von Faktor VIII**

Die Bestimmung von Faktor VIII-Antigen in FEIBA wurde nach der Methode von Moritz B. al. (Thromb. Haemost. 1997, Suppl:31) durchgeführt. Bei dieser Bestimmung wird durch einen monoklonalen Antikörper gegen die leichte Kette des Faktor VIII-Moleküls als Capture-Antikörper und mittels eines, ebenfalls gegen die leichte Kette des Faktor VIII-Moleküls, aber gegen ein anderes Epitop gerichteten, monoklonalen Antikörpers als Detektionsantikörper Faktor VIII selektiv neben anderen Plasmaproteinen in FEIBA nachgewiesen.

**Beispiel 2 : Bestimmung von Phospholipid**

Organisch gebundenes Phosphat wird aus dem lyophilisierten Pulver der FEIBA-Fraktion nach der Methode von Folch J. et al. (J. Biol.Chem. 1957,226:497-509) durch ein Lösungsmittelgemisch bestehend aus Chloroform, Methanol im Verhältnis von 2 Volumsteilen Chloroform zu 1 Volumsteil Methanol, extrahiert. Der den gesamten Phospholipidanteil enthaltende Extrakt wurde anschließend in Teflongefäße überführt und die organischen Lösungsmittel unter dem Stickstoffstrom abgedampft. Nach Zusatz eines Puffers (20 mM Tris HCl, 150 mM NaCl, pH 7,4) und Oxisolvreagenz (Fa. Merck) wurden die Teflongefäße dicht verschlossen und bei 160° für 5 h digeriert. Das durch den Digeriervorgang freigesetzte Phosphat wurde quantitativ photometrisch als Molybdatkomplex nach der Methode von Ames B.N. (Methods in Enzymology 1966,8: 115-118) nachgewiesen.

**Beispiel 3: Herstellung eines Faktor VIII-bindenden Affinitätsträgers**

Ein CHO-Zellklon, der rekombinanten von Willebrand-Faktor produziert, wird wie in FEBS.Lett. 1994; 351: 345-348, beschrieben, hergestellt. Durch Transfektion mit einem für die cDNA humanen Furins kodierenden Vektor (van den Ouweland et al., Nucleic Acids Res. 1990; 18:664) wurde die Zellinie zur Co-Expression von humanem Furin gebracht. Derartige stabile Zellklone wurden im Großmaßstab in Perfusionsreaktoren auf Microcarriern fermentiert (Blüml et al., in: Spier RE, Griffith JB, Berthold W, eds. Animal cell technology. Oxford, London: Butterworth-Heinemann, 1994:267-269).

Die Reinigung wurde durch ein 2-stufiges chromatographisches Verfahren gemäß Thromb.Haemost. 1995; 73:1160, durchgeführt. Die durch Elution mit Kochsalz desorbierte Fraktion wurde gewonnen und durch Gelfiltration über Sephadex G25 (Fa. Pharmacia) in einen Puffer, enthaltend 20 mM Tris-HCl, 150 mM NaCl, pH 7,5, umgepuffert. Anschließend wurde die Präparation durch Ultrakonzentration über eine Amicon YM30-Membran (cut-off: 30.000 D) auf eine Proteinkonzentration von 3 mg/ml aufkonzentriert. Die von Willebrand-Faktor-Konzentration in dieser Präparation betrug 60 E vWF-Antigen/mg Protein.

Die Präparation des rekombinanten von Willebrand-Faktors wurde mit einem Puffer, enthaltend 20 mM Tris-HCl, 150 mM NaCl, pH 7,5, auf 1,5 mg/ml verdünnt. Ein voraktiviertes, für die Affinitätschromatographie geeignetes Gel (Actigel, ALD-Superflow, Fa. Sterogene) wurde mit einem Puffer, enthaltend 20 mM Tris-HCl, 150 mM NaCl, pH 7,5, exzessiv vorgewaschen. Ein Volumsteil des vorgewaschenen Gels wurde mit 1,1 Volumsteilen der zu immobilisierenden Proteinlösung versetzt und anschließend 0,15 Volumsteile einer Lösung von 0,1 M Cyanborhydrid (NaCNBH3) in 0,1 M Phosphatpuffer, pH 7,0, zugesetzt. Das Gel wurde durch Schütteln in diesem Puffer suspendiert und unter weiterem Schütteln 16 Stunden bei Raumtemperatur inkubiert. Anschließend wurde das Gel auf einer Sinternutsche mit dem 10-fachen Volumen eines Puffers, enthaltend 20 mM Tris-HCl, 150 mM NaCl, pH 7,5, und mit dem 5-fachen Volumen eines Puffers, enthaltend 20 mM Tris-HCl, 2 M NaCl, pH 7,5, gewaschen. Dann wurde nochmals mit 5 Volumsteilen des Puffers, 20 mM Tris-HCl, 150 mM NaCl, pH 7,5, äquilibriert und das Gel in eine chromatographische Säule mit einer Dimension Durchmesser zu Gelbetthöhe von 1 : 4 übergeführt. Durch Bestimmung der Proteinkonzentration in den auf der Sinternutsche abgetrennten Lösungen des Inkubationsüberstandes der von Willebrand Faktor-Lösung und des Affinitätsgels sowie der Waschlösungen konnte eine Kopplungsrate von über 90 % des eingesetzten Proteins ermittelt werden.

**Beispiel 4: Herstellung von Faktor VIII-freiem Prothrombinkomplex durch Kontakt mit einem Affinitätsgel (Derzeit nach Ansicht der Anmelderin der beste Weg zur Ausführung der Erfindung) :**

Das pharmazeutische Präparat FEIBA S-TIM4 IMMUNO (1000 Einheiten) wurde mit 20 ml destilliertem Wasser rekonstituiert. Nach vollständiger Lösung des gefriergetrockneten Pulvers, enthielt die Lösung eine Wirkstoffkonzentration von 50 Einheiten pro ml. 10 ml dieser Lösung wurden mit 100 mg des vorher beschriebenen immobilisierten von Willebrand-Faktors in Kontakt gebracht und 1 h bei Raumtemperatur unter leichtem Schütteln inkubiert. Anschließend wurde das Immobilisat durch Filtration über eine Sinternutsche entfernt. Die im Filtrat erhaltene FEIBA-Lösung wies eine Aktivität von 49 Einheiten FEIBA/ml auf. Durch Bindung des im Präparat enthaltenen Faktor VIII an immobilisierten von Willebrand-Faktor, war Faktor VIII Antigen in dieser Präparation unter der Nachweisgrenze.

**Beispiel 5: Herstellung von Faktor VIII-freiem aktivierten Prothrombinkomplex durch Readsorption an einen unspezifischen Träger**

15 mg DEAE-Sephadex® A-50, Fa. Pharmacia, wurden 15 min. bei Raumtemperatur mit 1 ml einer Lösung von 30 g/l NaCl in Wasser zur Quellung inkubiert. Danach wurde das Gel durch Zentrifugation vom Quellüberstand abgetrennt. Anschließend folgten fünf Waschungen des Gels mit je 1 ml Puffer (9 g/l $Na_2HPO_4.2H_2O$, 7 g/l NaCl, pH 7,0) und weitere zwei Waschungen mit einem Puffer (7 g/l $Na_3$ Citrat. $2H_2O$, 7 g/l NaCl) ebenfalls durch Resuspendieren und Zentrifugieren.

30 ml frisch gefrorenes menschliches Citratplasma wurden bei 0 - +4°C aufgetaut und das anfallende Kryopräzipitat durch Zentrifugation bei +2°C abgetrennt. Der daraus resultierende "Kryoüberstand" wurde mit dem gewaschenen DEAE-Sephadex® inkubiert, wobei FEIBA generiert und zusammen mit den Faktoren des Prothrombinkomplexes, Faktor VIII und inertem Protein an das Gel adsorbiert wurde. Danach wurde coadsorbiertes Inertprotein vom DEAE-Gel durch Waschen mit einem Puffer (9 g/l $Na_2HPO_4.2H_2O$, 7 g/l NaCl) entfernt.

Der pufferfeuchte Gel-Proteinkomplex wurde nun mit 1,5 ml einer Lösung von 150 mg/ml TWEEN® -80 und 30 mg/ml NaCl 1 h bei 26°C suspendiert. Durch die Behandlung mit der Lösung hoher Ionenstärke wurde Protein gemeinsam mit den Faktoren des Prothrombinkomplexes und Faktor VIII desorbiert. Anschließend wurde die Suspension durch Zugabe von 6,5 ml Wasser verdünnt und 1 h bei Raumtemperatur readsorbiert, wobei die Prothrombinkomplexfraktion neuerlich readsorbiert wurde. Gleichzeitig wurde nur ein geringer Teil des in der Proteinfraktion enthaltenen Faktor VIII an das Gel readsorbiert. Der Gel/Proteinkomplex wurde dann fünf mal mit je 1 ml einer Lösung von 7 g/l NaCl in Wasser detergensfrei gewaschen.

Zur Elution wurde das Gel mit 0,7 ml einer Lösung von 30 g/l NaCl in Wasser unter Rühren behandelt. Das Eluat wurde nun gegen destilliertes Wasser dialysiert, eingefroren und lyophilisiert. Nach Rekonstitution des Lyophilisates wurde die FEIB-Aktivität gemäß AT 350 726 bestimmt.

Ferner wurden der Faktor VIII-Antigengehalt bestimmt. Als Kontrolle diente ein konventionell hergestelltes FEIBA-Präparat, wie es in AT 350 726 beschrieben ist. Die nach dem beschriebenen Verfahren gewonnene Präparation wies einen um einen Faktor 10 verminderten Faktor VIII-Gehalt im Vergleich zu Kontrolle auf.

Durch den Kontakt mit dem Detergens wurden auch eventuell vorhandene Pathogene, insbesondere lipidumhüllte Viren, inaktiviert.

**Beispiel 6 : Herstellung von Faktor VIII-freiem und Phospholipid-freiem aktivierten Prothrombinkomplex durch Readsorption an einen unspezifischen Träger**

15 mg DEAE-Sephadex® A-50, Fa. Pharmacia, wurden 15 min. bei Raumtemperatur mit 1 ml einer Lösung von 30 g/l MaCl in Wasser zur Quellung inkubiert. Danach wurde das Gel durch Zentrifugation vom Quellüberstand abgetrennt. Anschließend folgten fünf Waschungen des Gels mit je 1 ml Puffer (9 g/l $Na_2HPO_4.2H_2O$, 7 g/l NaCl, pH 7,0) und weitere zwei Waschungen mit einem Puffer (7 g/l $Na_3Citrat.2H_2O$, 7 g/l NaCl) ebenfalls durch Resuspendieren und Zentrifugieren.

30 ml frisch gefrorenes menschliches Citratplasma wurden bei 0 - +4°C aufgetaut und das anfallende Kryopräzipitat durch Zentrifugation bei +2 °C abgetrennt. Der daraus resultierende "Kryoüberstand" wurde mit dem gewaschenen DEAE-Sephadex® inkubiert, wobei FEIBA generiert und zusammen mit den Faktoren des Prothrombinkomplexes, Faktor VIII, Phospholipid und inertem Protein an das Gel adsorbiert wurde. Danach wurde coadsorbiertes Inertprotein vom DEAE-Gel durch Waschen mit einem Puffer (9 g/l $Na_2HPO_4.2H_2O$, 7 g/l NaCl) entfernt.

Der pufferfeuchte Gel-Proteinkomplex wurde nun mit 1,5 ml einer Lösung von 1 mg/ml TWEEN® -80 und 30 mg/ml NaCl 1 Stunde bei Raumtemperatur suspendiert, wobei die Proteinfraktion und unspezifisch adsorbierte Verunreinigungen desorbiert wurden. Anschließend wurde das Gel durch Filtration abgetrennt. Die Proteinlösung wurde nun durch weiteren Zusatz von TWEEN® -80 auf eine Detergenskonzentration von 150 mg/ml gebracht und anschließend 1 Stunde bei 40°C unter Rühren inkubiert. Anschließend wurde durch die Zugabe von 6,5 ml Wasser verdünnt und ein frisch gewaschenes vorbereitetes DEAE-Sephadex® A-50 Gel readsorbiert, wobei die Prothrombinkomplexfraktion neuerlich readsorbiert wurde. Gleichzeitig wurde nur ein geringer Teil des in der Proteinfraktion enthaltenen Faktor VIII an das Gel gebunden. Anschließend wurde durch fünf Waschungen mit je 1 ml einer Lösung von 7 g/l NaCl in Wasser detergensfrei gewaschen. Dabei wurde auch gebundenes Phospholipid, welches durch den Kontakt mit dem Amphiphil solubilisiert vorlag, entfernt.

Zur Elution wurde das Gel mit 0,7 ml einer Lösung von 30 g/l NaCl in Wasser unter Rühren behandelt. Das Eluat wurde nun gegen destilliertes Wasser dialysiert, eingefroren und lyophilisiert. Nach Rekonstitution des Lyophilisates wurde die FEIB-Aktivität gemäß der AT-B 350 726 bestimmt.

Ferner wurden der Faktor VIII-Antigengehalt und Phospholipid wie beschrieben bestimmt. Als Kontrolle diente ein konventionell hergestelltes FEIBA-Präparat, welches gemäß AT 350 726 gewonnen wurde. Die nach dem beschriebenen Verfahren erhaltene Präparation wies einen im Vergleich zur Kontrolle verminderten F VIII Gehalt auf und war frei von Phospholipid. Die Analysenergebnisse sind in Tabelle 1 zusammengefaßt.

Tabelle 1

| FEIBA: FVIII/Phospholipid-Gehalt | | | | |
|---|---|---|---|---|
| | FEIBA | FVIII C:Ag | $\frac{FVIII\ C:Ag}{FEIBA}$ | Phospholipid |
| | U/ml | U/ml | U/U | nM Phosphat/U FEIBA |
| FEIBA (Standard) | 51 | 7.0 | 0.14 | 0.14 |
| FEIBA (erfindungsgemäßes Produkt) | 70 | 1.0 | 0.01 | < 0.02 |

**Patentansprüche**

1.  Immuntolerante pharmazeutische Prothrombinkomplex-Präparation enthaltend die Faktoren II, IX, X und gegebenenfalls VII, dadurch gekennzeichnet, daß sie einen Faktor VIII-Antigen-Gehalt von weniger als 0,1 Faktor VIII:C-Antigen/E FEIBA aufweist.

2.  Pharmazeutische Präparation nach Anspruch 1, dadurch gekennzeichnet, daß sie aus Plasma oder einer Plasmafraktion hergestellt ist.

3.  Pharmazeutische Präparation nach Anspruch 1 oder 2, dadurch gekennzeichnet, daß die Präparation mindestens einen der Faktoren IXa, Xa und VIIa enthält und FEIB-Aktivität aufweist.

4.  Pharmazeutische Präparation nach einem der Ansprüche 1 bis 3, dadurch gekennzeichnet, daß sie frei von Phospholipiden ist.

5.  Verfahren zur Herstellung einer Prothrombinkomplex-Präparation umfassend die folgenden Schritte:

    a) zur Verfügung stellen von Plasma oder einer Plasmafraktion enthaltend die Faktoren II, IX, X und gegebenenfalls VII,
    b) in Kontakt bringen des Plasmas oder der Plasmafraktion mit einem Trägermaterial, gegebenenfalls in Gegenwart eines Detergens, so daß Faktor VIII und gegebenenfalls Phospholipide von den Faktoren II, IX, X und gegebenenfalls VII getrennt werden,
    c) reinigen des Plasmas oder der Plasmafraktion, und
    d) gewinnen einer Fraktion enthaltend die Faktoren II, IX, X und gegebenenfalls VII.

6.  Verfahren nach Anspruch 5, dadurch gekennzeichnet, daß die Schritte b und c als ein Vertahrensschritt durchgeführt werden.

7.  Verfahren nach Anspruch 5 oder 6, dadurch gekennzeichnet, daß das Trägermaterial ein für die Chromatographie, Filtration und/oder Nanofiltration geeignetes Material ist.

**Claims**

1.  An immunotolerant pharmaceutical prothrombin complex preparation comprising factors II, IX, X and, optionally, VII, characterised in that it has a factor VIII-antigen content of less than 0.1 factor VIII:C antigen/U FEIBA.

2.  A pharmaceutical preparation according to claim 1, characterised in that it is prepared from plasma or from a plasma fraction.

3.  A pharmaceutical preparation according to claim 1 or 2, characterised in that the preparation comprises at least one of factors IXa, Xa and VIIa and has FEIB activity.

4.  A pharmaceutical preparation according to any one of claims 1 to 3, characterised in that it is free from phospholipids.

5.  A method of preparing a prothrombin-complex preparation comprising the following steps:

    a) providing plasma or a plasma fraction comprising factors II, IX, X and, optionally, VII,
    b) contacting the plasma or the plasma fraction with a carrier material, optionally in the presence of a detergent, so that factor VIII and optionally phospholipids are separated from the factors II, IX, X and, optionally, VII,
    c) purifying the plasma or the plasma fraction, and
    d) recovering a fraction comprising factors II, IX, X and, optionally, VII.

6.  A method according to claim 5, characterised in that steps b and c are carried out as one method step.

7.  A method according to claim 5 or 6, characterised in that the carrier material is a material suitable for chromatography, filtration and/or nanofiltration.

**Revendications**

1. Préparation pharmaceutique immunotolérante d'un complexe de prothrombine contenant les facteurs II, IX, X et éventuellement VII, caractérisée en ce qu'il a une teneur en l'antigène du facteur VIII inférieure à 0,1 facteur VIII : antigène C/E FEIBA.

2. Préparation pharmaceutique selon la revendication 1, caractérisée en ce qu'elle est préparée à partir de plasma ou d'une fraction de plasma.

3. Préparation pharmaceutique selon la revendication 1 ou 2, caractérisée en ce que la préparation contient au moins l'un des facteurs IXa, Xa et VIIa, et a une activité de FEIB.

4. Préparation pharmaceutique selon l'une des revendications 1 à 3, caractérisée en ce qu'elle est exempte de phospholipides.

5. Procédé de préparation d'une préparation d'un complexe de prothrombine, qui comprend les étapes suivantes :

   a) mise à disposition de plasma ou d'une fraction de plasma contenant les facteurs II, IX, X et éventuellement VII,
   b) mise en contact du plasma ou de la fraction de plasma avec un matériau support, éventuellement en présence d'un détergent, de sorte que le facteur VIII et éventuellement les phospholipides soient séparés des facteurs II, IX, X et éventuellement VII,
   c) purification du plasma ou de la fraction de plasma, et
   d) obtention d'une fraction contenant les facteurs II, IX, X et éventuellement VII.

6. Procédé selon la revendication 5, caractérisé en ce que les étapes b) et c) sont mises en oeuvre sous forme d'une étape unique.

7. Procédé selon la revendication 5 ou 6, caractérisé en ce que le matériau support est un matériau convenant à une chromatographie, à une filtration et/ou à une nanofiltration.